## Europäisches Patentamt
## European Patent Office
## Office européen des brevets

(19)

(11) Veröffentlichungsnummer: **0 030 355**
**B1**

(12) **EUROPÄISCHE PATENTSCHRIFT**

(45) Veröffentlichungstag der Patentschrift:
31.10.84

(51) Int. Cl.³: **A 61 B 6/00**, A 61 B 19/00

(21) Anmeldenummer: 80107532.6

(22) Anmeldetag: 02.12.80

(54) **Medizinische Untersuchungsanlage.**

(30) Priorität: 05.12.79 DE 2948986

(43) Veröffentlichungstag der Anmeldung:
17.06.81 Patentblatt 81/24

(45) Bekanntmachung des Hinweises auf die Patenterteilung:
31.10.84 Patentblatt 84/44

(84) Benannte Vertragsstaaten:
FR GB IT

(56) Entgegenhaltungen:
EP - A - 0 018 166
DE - A - 2 115 121
DE - A - 2 809 645

Fachlexicon ABC Physik Verlag Harri Deutsch, 1974,
Seite 1135

(73) Patentinhaber: Siemens Aktiengesellschaft, Berlin und
München Wittelsbacherplatz 2, D-8000 München 2 (DE)

(72) Erfinder: Bär, Ulrich, Hermann-Köhl-Weg 15,
D-8500 Nürnberg (DE)
Erfinder: Huk, Walter, Dr., Fliessbachstrasse 14,
D-8520 Erlangen (DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Beschreibung

Die Erfindung betrifft eine medizinische Untersuchungsanlage mit einem Computertomographen für Transversalschichtbilder mit Fixierungsmitteln für den Patienten.

In der modernen Röntgendiagnostik werden in zunehmendem Maße Computertomographen eingesetzt, bei denen der Patient durch eine aus einer Röntgenstrahlenquelle und einem Strahlenempfänger bestehende Meßanordnung in einer Transversalschicht abgetastet wird. Aus den vom Strahlenempfänger gelieferten Meßwerten, die der Schwächung der Röntgenstrahlung im Patienten entsprechen, berechnet ein Computer ein Bild der abgetasteten Transversalschicht. Ein wesentliches Anwendungsgebiet eines Computertomographen ist die Untersuchung des Schädels, z. B. zur Erfassung von Tumoren. Wird ein Tumor im Röntgenbild festgestellt, so ist es erforderlich, eine Gewebeprobe aus dem Tumorgewebe zu entnehmen. Dabei ergibt sich das Problem, eine Biopsienadel durch eine Knochenlücke im Schädel gezielt so zu führen, daß sie genau die Stelle des Gewebes erreicht, an der eine Gewebeprobe entnommen werden soll.

Es ist bereits ein Gerät für stereotaktische Gehirnoperationen bekannt, bei dem Fixierungsmittel für den Patienten sowie eine Patienten-Zielvorrichtung mit Mitteln zum Einführen in den Patienten, die durch die Zielvorrichtung ausrichtbar sind, vorhanden sind. Bei diesem Gerät werden die Einstellungswerte der Zielvorrichtung zunächst an einem Phantomring ermittelt und dann auf das eigentliche Operationsgerät übertragen (DE-A-2 115 121).

Dieser Schrift läßt sich jedoch nicht entnehmen, wie die Einstellung der Zielvorrichtung am Phantomring im einzelnen erfolgen soll. Insbesondere ergibt sich daraus nicht, wie bei Anwendung des Gerätes in Verbindung mit einem Computertomographen die Einstellung des Phantomringes im einzelnen vorgenommen werden soll.

Der Erfindung liegt die Aufgabe zugrunde, eine medizinische Untersuchungsanlage der eingangs genannten Art so auszubilden, daß das Phantomgerät in einfacher Weise den vom Computertomographen erhaltenen Daten entsprechend einstellbar ist.

Diese Aufgabe ist erfindungsgemäß durch die im Kennzeichen des Patentanspruchs angegebene Ausbildung gelöst. Bei der erfindungsgemäßen Untersuchungsanlage kann der Patient unverrückbar in die Fixierungsmittel eingespannt werden. Mit Hilfe der Patientenzielvorrichtung und einer Computertomographie-Aufnahme ist eine Einstellung des Phantomgerätes entsprechend den Gegebenheiten möglich. Die Simulations-Zielvorrichtung kann dann auf eine bestimmte Körperstelle ausgerichtet und ihre Ausrichtung auf die Patientenzielvorrichtung übertragen werden. Es ist aber auch möglich, die Patientenzielvorrichtung am Phantomgerät auszurichten, von diesem Gerät zu entfernen und mit den Fixierungsmitteln für den Patienten zu verbinden, so daß nur eine einzige Zielvorrichtung erforderlich ist.

Eine Ausgestaltung der Erfindung besteht darin, daß jede Zielvorrichtung eine Halterung für eine Biopsienadel aufweist, die eine freie Einstellung der Biopsienadel im Raum erlaubt. Bei dieser Ausgestaltung ist die Entnahme von Gewebeproben, z. B. aus einem Tumorgewebe, mit Hilfe einer Biopsienadel möglich. Es ist ferner vorteilhaft, jedes Simulationsteil mit einer in einer Ebene eines Rades verstellbaren Marke zu versehen, und das Rad in Richtung seiner Achse verstellbar zu lagern. Dabei können z. B. zwei Räder am Phantomgerät vorgesehen sein, eines zur Simulation der Knochenlücke im Schädel eines Patienten zum Einführen einer Biopsienadel, und das andere zur Simulation der Ebene, in der eine Gewebeprobe entnommen werden soll.

Die Erfindung ist nachfolgend anhand eines in der Zeichnung dargestellten Ausführungsbeispiels näher erläutert. Es zeigt

Fig. 1 einen Computertomographen mit Fixiermitteln und einer Patientenzielvorrichtung einer Untersuchungsanlage nach der Erfindung,

Fig. 2 eine Ansicht der Zielvorrichtung nach Fig. 1,

Fig. 3 die Zielvorrichtung nach Fig. 1 und 2 von oben,

Fig. 4 ein Phantomgerät einer Untersuchungsanlage nach der Erfindung,

Fig. 5 bis 7 Einzelheiten des Phantomgerätes nach Fig. 4 und

Fig. 8 bis 12 Bilder zur Erläuterung der Einstellung der Zielvorrichtung sowie

Fig. 13 bis 15 Varianten der Fixiermittel, die besonders zweckmäßig sind.

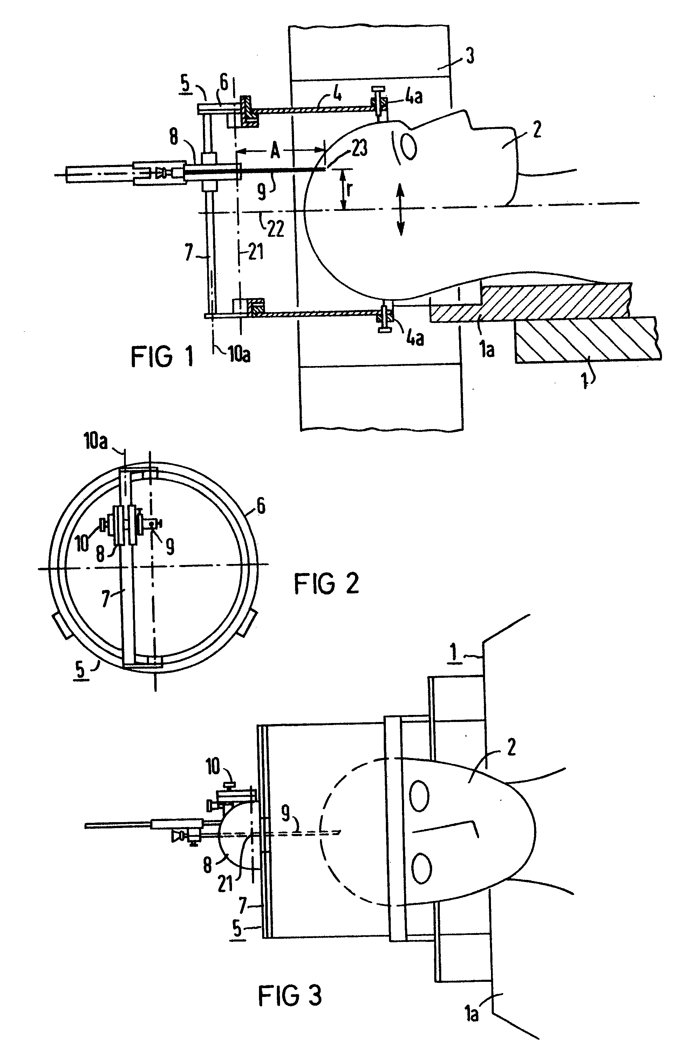

In den Fig. 1 und 3 ist eine Patientenlage 1 dargestellt, auf der eine Lagerungsplatte 1a mit einem Patienten 2 ruht. Der Kopf des Patienten 2 ist in der Öffnung eines schematisch dargestellten Computertomographen 3 mit Hilfe von Fixiermitteln 4 fixiert. Die Fixiermittel 4 bestehen aus Schrauben in einem Ring 4a, die am Kopf des Patienten 2 anliegen und diesen festhalten. Der Ring 4a mit einer Patientenzielvorrichtung 5 ist gegenüber der Lagerungsplatte 1a höhenverstellbar. Die Patientenzielvorrichtung 5 ist mit den Fixiermitteln 4 verbunden und weist gemäß Fig. 2 an einem ringförmigen Rahmen 6 einen Bügel 7 auf, der längsverschiebbar einen Halter 8 für eine Biopsienadel 9 trägt. Die Biopsienadel 9 ist am Halter 8 um zwei zueinander senkrechte Achsen 10, 10a schwenkbar gelagert und daher frei im Raum einstellbar.

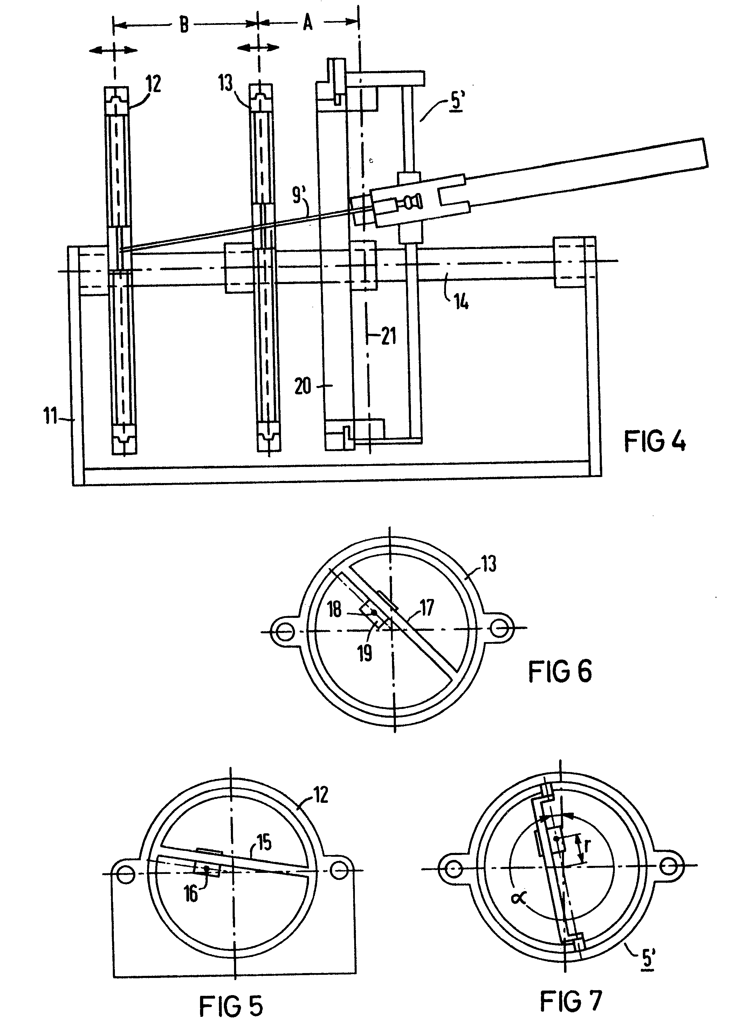

Das Phantomgerät gemäß den Fig. 4 bis 7 weist auf einem Sockel 11 Simulationsteile für bestimmte Körperstellen auf, die von Rädern 12, 13 gebildet sind. Die Räder 12, 13 sind auf einer Achse 14 gelagert. Das Rad 12 trägt gemäß Fig. 5 eine auf einer Speiche 15 längsverschiebbare, also in seiner Ebene längs eines Durchmessers verstellbare Marke 16. Das Rad 13 trägt analog dazu eine auf einer Speiche 17 längs eines Durchmessers verstellbare Marke 18, die von einem Loch in einer Platte 19 gebildet ist. Auf der Achse 14 ist ein Halter 20 gelagert, der eine Zielvorrichtung 5' trägt, die der Zielvorrichtung 5 der Fig. 1 bis 3 entspricht.

Für die Entnahme einer Gewebeprobe mittels der Biopsienadel 9, die auf der Zielvorrichtung 5 frei einstellbar ist, geht man folgendermaßen vor:

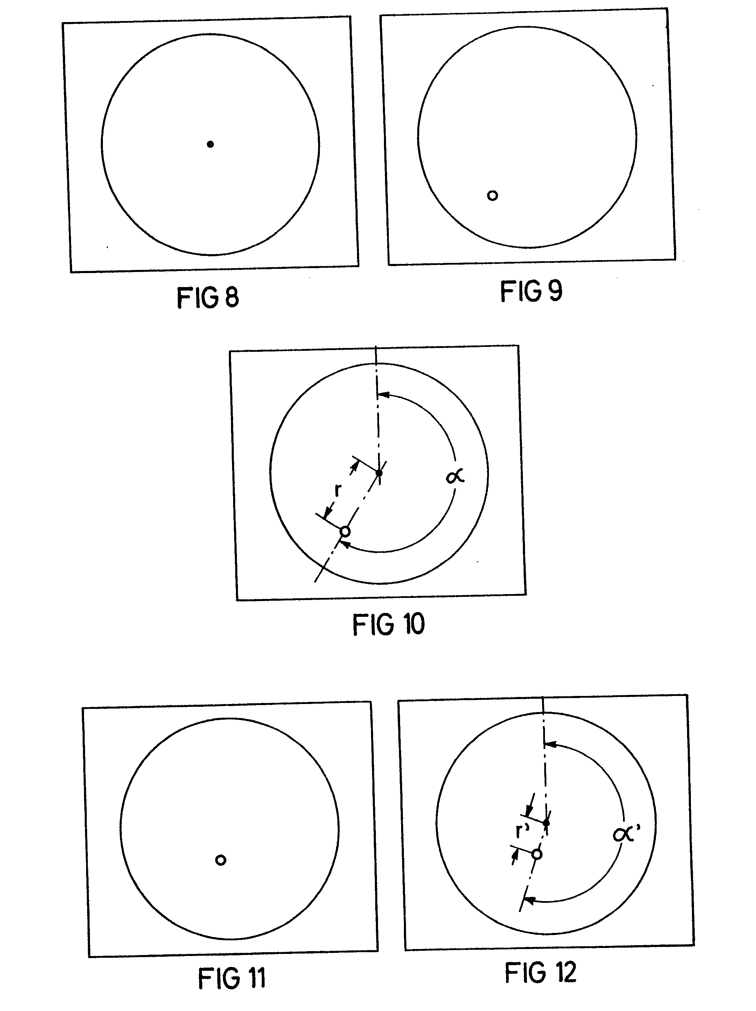

Die Biopsienadel 9 der Zielvorrichtung 5 wird in ihre Null-Lage gestellt, in der sie in der Mittelachse 22 der Zielvorrichtung 5 liegt. Anschließend wird mittels des Computertomographen 3, der beispielsweise gemäß der DE-OS 2 438 708 ausgebildet sein kann, eine Aufnahme der Biopsienadel 9 angefertigt, die in der Fig. 8 dargestellt ist. Die Biopsienadel 9 markiert in dieser Aufnahme den Bezugsmittelpunkt, der für die weitere Auswertung von Bedeutung ist. Nach dieser Aufnahme darf das Gerät nicht mehr verrückt werden.

Zunächst wird das Maß A, der Abstand zwischen der Bezugsebene 21 der Zielvorrichtung 5 und der Knochenlücke 23 im Schädel des Patienten 2 mit der Zielvorrichtung 5 ermittelt. Das Maß A wird am Phantomgerät gemäß Fig. 4 durch Verschieben des Rades 13 eingestellt.

Anschließend wird mit dem Computertomographen 3 eine Aufnahme der Knochenlücke 23 im Kopf des Patienten 2 angefertigt. Diese Aufnahme ist in der Fig. 9 dargestellt. Sie zeigt die Transversalschicht des Kopfes des Patienten 2, in der die Knochenlücke 23 liegt.

Die Bilder gemäß den Fig. 8 und 9 werden überlagert und ergeben ein Bild gemäß Fig. 10. Aus dem Bild gemäß Fig. 10 wird der Winkel $\alpha$ und der Abstand r ermittelt und diese Größen werden mit Hilfe der Marke 18 am Rad 13 eingestellt.

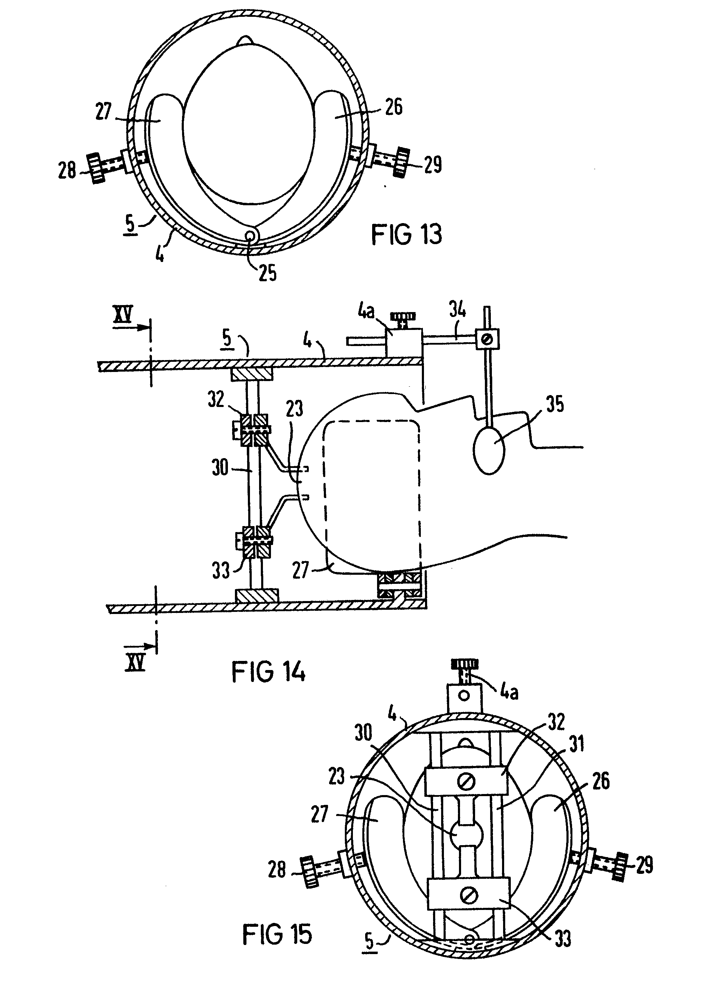

Nunmehr wird mittels des Computertomographen 3 diejenige Transversalschicht abgebildet, in der der Zielpunkt, beispielsweise ein Tumor, liegt. Man erhält ein Bild gemäß der Fig. 11. Bei dieser Aufnahme muß der Wert B, nämlich der Abstand zwischen der Transversalschicht, in der die Knochenlücke 23 liegt und der Transversalschicht, in der der Zielpunkt liegt, abgelesen und im Phantomgerät gemäß Fig. 4 durch Verschieben des Rades 12 eingestellt werden.

Werden die Bilder gemäß Fig. 8 und 11 überlagert, so erhält man das Bild gemäß Fig. 12. Aus diesem Bild wird der Winkel $\alpha'$ und der Abstand r' ermittelt und die Marke 16 des Rades 12 entsprechend diesen Größen eingestellt.

Die Biopsienadel 9' des Phantomgerätes wird nunmehr so eingestellt, daß sie durch die Marke 18 des Rades 13 verläuft und auf die Marke 16 des Rades 12 zeigt.

Nunmehr ergeben sich zwei Möglichkeiten. Für den Fall, daß die Zielvorrichtungen 5, 5' zwei getrennte Zielvorrichtungen sind, werden die an der Zielvorrichtung 5' eingestellten Werte auf die Zielvorrichtung 5 übertragen. Nach dieser Übertragung hat die Biopsienadel 9 die richtige Lage und ihre Spitze liegt an der Stelle, an der Gewebe entnommen werden soll. Die zweite Möglichkeit ist, nur eine einzige Zielvorrichtung vorzusehen, und diese Zielvorrichtung nach Einstellung vom Phantomgerät zu entfernen und mit der Fixiervorrichtung 4 zu verbinden. Dabei müssen natürlich die am Phantomgerät eingestellten Werte arretiert werden.

Die in den Fig. 13 bis 15 dargestellten Fixiermittel weisen zwei durch ein Scharnier 25 gelenkig miteinander verbundene Polster 26, 27 auf, die auf starren Basisplatten befestigt sind und die durch Schrauben 28, 29 an den Kopf des Patienten andrückbar sind. Zur weiteren Fixierung sind zwei auf Führungen 30, 31 längsverschiebbare und arretierbare Klammern 32, 33 vorgesehen, die in die Knochenlücke 23 eingeführt und dort verspannt werden. Ferner ist an einem Halter 34 ein Polster 35 befestigt, das in dem Mund des Patienten eingeführt wird und den Oberkiefer fixiert. Der Halter 34 ist im Ring 4a verschiebbar und arretierbar.

## Patentanspruch

Medizinische Untersuchungsanlage mit einem Computertomographen (1, 3) für Transversalschichtbilder mit Fixierungsmitteln (4) für den Patienten (2), gekennzeichnet durch eine Patienten-Zielvorrichtung (5) mit einer Biopsienadel (9) zum Einführen in den Patienten (2), die durch die Zielvorrichtung (5) ausrichtbar ist, und ein Phantomgerät (Fig. 4) mit verstellbaren Simulationsteilen (12, 13, 15 bis 19) für bestimmte Körperstellen sowie einer auf die Simulationsteile (12, 13, 15 bis 19) ausrichtbaren Simulations-Zielvorrichtung (5') oder Mitteln (20) zum Verbinden der Patienten-Zielvorrichtung (5) mit dem Phantomgerät (Fig. 4), bei dem jedes Simulationsteil (12, 13, 15 bis 19) eine längs eines Durchmessers eines Rades (12, 13) verstellbare Marke (16, 18) aufweist und bei dem das Rad (12, 13) in Richtung einer Achse (14) verstellbar gelagert ist.

## Claim

Medical examination apparatus comprising a computertomograph (1, 3) for transverse layer images having fixing means (4) for the patient (2), characterised by a patient-target device (5) having a biopsy needle (9) for inserting into the patient (2) which can be aligned by means of the target device (5), and a phantom apparatus (fig. 4) having adjustable simulation components (12, 13, 15 to 19) for specific parts of the body and a simulation-target device (5'), which can be aligned with the simulation components (12, 13, 15 to 19), or means (20) for connecting the patient-target device (5) to the phantom apparatus (fig. 4), each simulation component (12, 13, 15 to 19) having a mark which is adjustable along a diameter of a wheel (12, 13), and the wheel (12, 13) being supported so as to be adjustable in the direction of an axle (14).

## Revendication

Installation pour l'examen médical à l'aide d'un tomodensitomètre (1, 3) pour la tomographie transversale avec des moyens de fixation (4) pour le patient, caractérisée par un dispositif de visée (5) du patient avec une aiguille biopsique (9) pour l'introduction dans le patient (2) et qui est orientable à l'aide du dispositif de visée (5), et un appareil fantôme (fig. 4) à éléments de simulation réglables (12, 13, 15 à 19) pour des parties déterminées du corps, ainsi qu'avec un dispositif de visée de simulation (5') orientable sur les éléments de simulation (12, 13, 15 à 19) au des moyens (20) pour relier le dispositif de visée du patient (5) avec l'appareil fantôme (fig. 4), dans lequel chaque élément de simulation (12, 13, 15 à 19) comporte un repère (16, 18) déplaçable le long d'un diamètre d'une roue (12, 13), et dans lequel la roue (12, 13) est montée de façon à être déplaçable en direction d'un axe (14).

FIG 1

FIG 2

FIG 3

B

A

12

13

5'

9'

14

21

20

11

FIG 4

13

18  17

19

FIG 6

12

15

16

FIG 5

r

α

5'

FIG 7

7

FIG 8

FIG 9

FIG 10

FIG 11

FIG 12

FIG 13

FIG 14

FIG 15